(19) **Europäisches Patentamt / European Patent Office / Office européen des brevets**

(11) **EP 4 245 836 A1**

(12) # EUROPEAN PATENT APPLICATION
published in accordance with Art. 153(4) EPC

(43) Date of publication:
**20.09.2023 Bulletin 2023/38**

(21) Application number: **21891935.5**

(22) Date of filing: **11.11.2021**

(51) International Patent Classification (IPC):
*C12M 1/00* (2006.01)     *C12M 1/04* (2006.01)
*C12M 1/34* (2006.01)     *C12M 3/00* (2006.01)
*C12Q 1/02* (2006.01)

(52) Cooperative Patent Classification (CPC):
**C12M 1/00; C12M 1/04; C12M 1/34; C12M 3/00; C12Q 1/02**

(86) International application number:
**PCT/JP2021/041461**

(87) International publication number:
**WO 2022/102690 (19.05.2022 Gazette 2022/20)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **13.11.2020 JP 2020189108**

(71) Applicant: **Toyo Seikan Group Holdings, Ltd.**
**Shinagawa-ku**
**Tokyo 141-8627 (JP)**

(72) Inventors:
• **KOSEKI, Osamu**
  **Yokohama-shi, Kanagawa 240-0062 (JP)**
• **TANAKA, Satoshi**
  **Yokohama-shi, Kanagawa 240-0062 (JP)**
• **TOTANI, Takahiko**
  **Yokohama-shi, Kanagawa 240-0062 (JP)**
• **MATSUOKA, Yosuke**
  **Yokohama-shi, Kanagawa 240-0062 (JP)**
• **NISHIYAMA, Takaharu**
  **Yokohama-shi, Kanagawa 240-0062 (JP)**

(74) Representative: **Hasegawa, Kan**
**Patentanwaltskanzlei Hasegawa**
**Untere Hauptstraße 56**
**85354 Freising (DE)**

(54) **CELL CULTURE SYSTEM AND METHOD OF DETECTING PROLIFERATIVE PROPERTIES OF CELLS**

(57)     Provided is a cell culture system capable of detecting a proliferative property of cells in a culture vessel at a desired timing during a culture when the cells are statically cultured in the culture vessel formed of a gas permeable member. A cell culture system is a system for statically culturing cells with a culture vessel 20 at least partially formed of a gas permeable member. The cell culture system includes an oxygen concentration sensor 21 that measures an oxygen concentration in a vicinity of a culture surface inside the culture vessel 20, and a proliferation detection unit 631 that detects a proliferative property of the cells based on an oxygen permeability of the gas permeable member, a measurement value of the oxygen concentration sensor, an oxygen concentration around the culture vessel 20, and an oxygen consumption per cell of the cells.

EP 4 245 836 A1

Fig. 1

**Description**

TECHNICAL FIELD

The present invention relates to a cell culture technique, and especially relates to a cell culture system that detects a proliferative property of cells when the cells are statically cultured with a culture vessel formed of a gas permeable member.

BACKGROUND ART

Recently, in the production of pharmaceutical medicines and in the fields of gene therapy, regeneration medicine, immunotherapy, and the like, it is desired that cells and tissues are efficiently cultured in a large amount under an artificial environment.

In such a situation, it has been proposed that cells are automatically cultured in large quantities in a closed system using a bag-shaped cell culture vessel formed of a gas permeable member.

When the cells are cultured in large quantities, it is important to confirm that the culture is properly performed by detecting the proliferative property of the cells. Additionally, for the detection of the proliferative property of cells, for example, it is preferred to allow confirming the number of cells in the culture vessel at desired timings during the culture.

As a method for confirming the number of cells in the culture vessel, the following three methods can be presented.

(1) A part of a medium inside the culture vessel is extracted (sampling), and the number of cells is measured using a hemocytometer or the like.
(2) A photograph of cells in the culture vessel is taken by a microscope and a camera, and image processing is performed on the obtained photograph to count the number of cells.
(3) The number of cells is counted based on the decreased amount of dissolved oxygen in the culture vessel.

CITATION LIST

Patent Literature

Patent Document 1: JP-A-S63-15150

Patent Document 2: JP-A-H6-121667

DISCLOSURE OF THE INVENTION

PROBLEMS TO BE SOLVED BY THE INVENTION

However, the method (1) has a problem that the risk of contamination increases because a part of the culture vessel is required to be opened for sampling the medium. Additionally, there is a problem that when adherent cells are cultured, it is impossible to detach only a part of the cells during the culture, and the number of cells cannot be counted at a desired timing.

The method (2) has a problem that when floating cells are cultured, while the measurement can be performed when the culture density is low, the measurement becomes difficult because of the lamination of the cells when the cell density increases after the middle of the culture. Additionally, there is also a problem that since this method requires the microscope, the camera, and an image processing device, the mechanism of the cell culture system is complicated.

Furthermore, in the method (3), since it is necessary to measure the decreased amount of the dissolved oxygen in the culture vessel, a sealed state is required for avoiding the move of a gas from the inside to the outside of the culture vessel, and the entering of a gas into the culture vessel from outside. Alternatively, when there is a space on a culture medium inside the culture vessel, it is necessary to interrupt dissolution of oxygen into the culture medium by replacing the space with nitrogen, and measure the consumption quantity of the dissolved oxygen in the culture medium by the cells.

However, there is a problem that when cells are cultured using a culture vessel formed of a gas permeable member, since it is impossible to detect the density change of only the dissolved oxygen consumed by the cells in the culture medium, and it is necessary to stir inside the culture vessel for the measurement, static culture cannot be performed.

Here, while Patent Document 1 discloses a method for measuring a decreased amount of dissolved oxygen in a sample solution containing microorganisms in a state where the sample solution is sealed within a container, this measuring method needs to be performed using a culture vessel without gas permeability.

Patent Document 2 discloses a cell culture device that operates an amount of temporal variation of oxygen amount relating to the number of cells in a culture vessel using an oxygen amount measured by oxygen amount measurement means that measures an oxygen amount dissolved in a medium in the culture vessel. However, this cell culture device uses a sealing container that avoids entering of a gas into a culture vessel from outside, and requires measuring a consumption of dissolved oxygen in a culture medium by changing a culture condition, for example, replacing a space on the culture medium inside the culture vessel with nitrogen to interrupt dissolution of oxygen into the culture medium, for obtaining a measurable state from an ordinary culture state.

The inventions disclosed in these patent documents cannot be appropriately used when cells are statically cultured with a culture vessel formed of a gas permeable member.

The present invention has been made in consideration of the above-described circumstances, and it is an object of the present invention to provide a cell culture system capable of detecting a proliferative property of cells in a culture vessel at a desired timing during a culture when the cells are statically cultured in the culture vessel formed of a gas permeable member, and a method for detecting the proliferative property of cells.

SOLUTIONS TO THE PROBLEMS

To achieve the above-described object, a cell culture system of the present invention is a cell culture system for statically culturing cells with a culture vessel.

The culture vessel is at least partially formed of a gas permeable member. The cell culture system includes an oxygen concentration sensor and a proliferation detection unit. The oxygen concentration sensor measures an oxygen concentration in a vicinity of a culture surface inside the culture vessel. The proliferation detection unit detects a proliferative property of the cells based on an oxygen permeability of the gas permeable member, a measurement value of the oxygen concentration sensor, an oxygen concentration around the culture vessel, and an oxygen consumption per cell of the cells.

In the cell culture system of the present invention, the proliferation detection unit preferably calculates a count of the cells per unit area as the proliferative property of the cells by a formula (1) below,

$$C = G \times (M/100 - D/100)/S \ldots \text{Formula (1)}$$

C: count of cells per unit area (cells/cm$^2$)
G: oxygen permeability of gas permeable member (mg/(cm$^2$·hr·atm))
M: oxygen concentration around culture vessel (%)
D: measurement value of oxygen concentration sensor (%)
S: oxygen consumption of one cell per unit time (mg/(hr cells)).

In the cell culture system of the present invention, the proliferation detection unit preferably uses an estimated value of an oxygen concentration inside the culture vessel at a desired time point as the measurement value of the oxygen concentration sensor. The estimated value of the oxygen concentration inside the culture vessel at a desired time point is preferably obtained based on a ratio between a slope of a change in rise peak value of the oxygen concentration when an additional medium is added in the culture vessel multiple times and a slope of a change of the oxygen concentration when the culture vessel is stabilized.

In the cell culture system of the present invention, the oxygen concentration around the culture vessel is preferably an oxygen concentration inside an incubator in which the culture vessel is housed, or an atmospheric oxygen concentration.

In the cell culture system of the present invention, the oxygen concentration sensor is preferably a fluorescent type.

In the cell culture system of the present invention, the oxygen concentration sensor is preferably secured to the culture surface inside the culture vessel using a perforated resin member.

A method for detecting a proliferative property of cells of the present invention is a method for detecting a proliferative property of cells in a cell culture in which the cells are statically cultured with a culture vessel. The culture vessel is at least partially formed of a gas permeable member. The method includes: measuring an oxygen concentration in a vicinity of a culture surface inside the culture vessel by an oxygen concentration sensor disposed on the culture surface inside the culture vessel; and calculating a count of the cells per unit area based on an oxygen permeability of the gas permeable member, a measurement value of the oxygen concentration sensor, an oxygen concentration around the culture vessel, and an oxygen consumption per cell of the cells.

EFFECTS OF THE INVENTION

The present invention can provide the cell culture system capable of detecting the proliferative property of the cells in the culture vessel at a desired timing during the culture when the cells are statically cultured in the culture vessel formed of the gas permeable member, and the method for detecting the proliferative property of the cells.

BRIEF DESCRIPTION OF THE DRAWINGS

FIG. 1 is a schematic diagram illustrating a configuration of a cell culture system according to an embodiment of the present invention.

FIG. 2 is an explanatory view illustrating a distribution of oxygen concentration inside a culture vessel in the cell culture system according to the embodiment of the present invention.

FIG. 3 is an explanatory view illustrating a secured state of an oxygen concentration sensor inside the culture vessel in the cell culture system according to the embodiment of the present invention.

FIG. 4 is a drawing illustrating a graph indicating a measurement result of an oxygen concentration by a fluorescent oxygen concentration sensor secured to a culture bag in a cell culture of Test 1.

FIG. 5 is drawing illustrating a graph indicating a measurement result of the oxygen concentration by the fluorescent oxygen concentration sensor secured to the culture bag in a cell culture of Test 2.

FIG. 6 is a drawing illustrating a graph (1) indicating a measurement result of the oxygen concentration by the fluorescent oxygen concentration sensor secured to the culture bag in a cell culture of Test 3.

FIG. 7 is a drawing illustrating a graph (2) indicating a measurement result of the oxygen concentration by the fluorescent oxygen concentration sensor secured to the culture bag in the cell culture of Test 3.

FIG. 8 is a schematic diagram illustrating a part of a configuration of a culture bag used in a cell culture of Test 4 in the cell culture system and the method for detecting proliferative property of cells according to the embodiment.

FIG. 9 is a drawing illustrating a graph indicating a measurement result of the oxygen concentration by the fluorescent oxygen concentration sensor secured to the culture bag in the cell culture of Test 4.

FIG. 10 is a drawing illustrating a graph indicating a measurement result of the oxygen concentration by the fluorescent oxygen concentration sensor secured to the culture bag in a cell culture of Test 5.

DESCRIPTION OF EMBODIMENTS

The following describes embodiments of a cell culture system and a method for detecting a proliferative property of cells of the present invention in detail. However, the present invention is not limited to the specific contents of the embodiments and examples below.

A cell culture system of this embodiment is a cell culture system for statically culturing cells with a culture vessel at least partially formed of a gas permeable member, and includes an oxygen concentration sensor that measures an oxygen concentration in a vicinity of a culture surface inside the culture vessel, and a proliferation detection unit that detects a proliferative property of cells based on an oxygen permeability of the gas permeable member, a measurement value of the oxygen concentration sensor, an oxygen concentration around the culture vessel, and an oxygen consumption per cell of the cells.

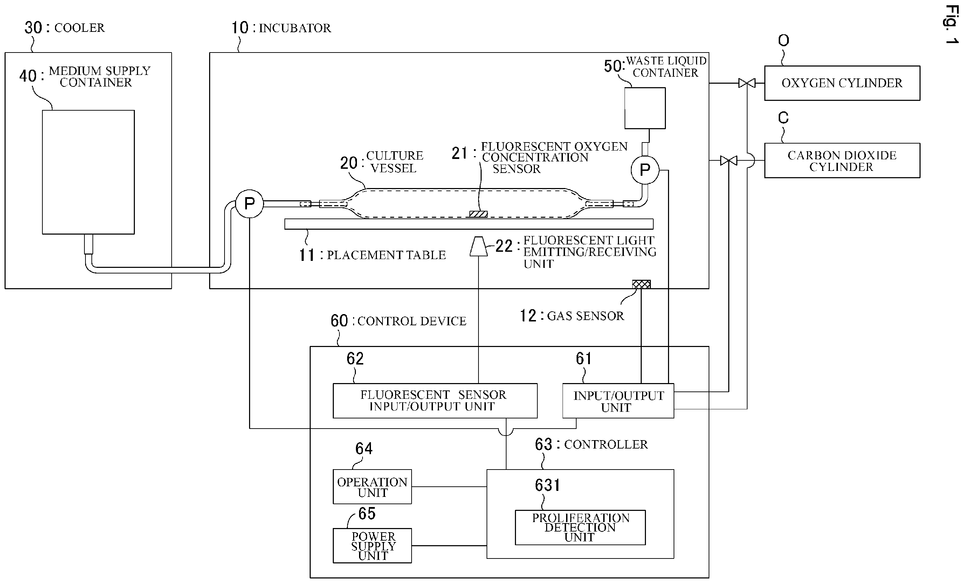

Specifically, the cell culture system of this embodiment can have, for example, a configuration as illustrated in FIG. 1.

First, a schematic configuration of the cell culture system of this embodiment will be described.

In the cell culture system of this embodiment, a culture vessel 20 is housed in an incubator 10, and placed on a placement table 11. The culture vessel 20 includes two ports, and a medium supply container 40 housed in a cooler 30 is coupled to one port by a tube. To the other port of the culture vessel 20, a waste liquid container 50 inside the incubator 10 is coupled by a tube.

At the tube coupling the culture vessel 20 to the medium supply container 40 and the tube coupling the culture vessel 20 to the waste liquid container 50, liquid transfer means is disposed. As the liquid transfer means, for example, a pump, such as a peristaltic pump and a syringe pump, capable of transferring a liquid at a low speed with high accuracy is preferred to be used.

Then, a control device 60 controls the liquid transfer means between the culture vessel 20 and the medium supply container 40 at a predetermined timing, thus transferring a medium from the medium supply container 40 to the culture vessel 20. The control device 60 controls the liquid transfer means between the culture vessel 20 and the waste liquid container 50 at a predetermined timing, thus transferring the medium from the culture vessel 20 to the waste liquid container 50.

By supplying an oxygen gas and a carbon dioxide gas from an oxygen cylinder O (oxygen supply device) and a carbon dioxide cylinder C (carbon dioxide supply device) to the incubator 10, respectively, a gas concentration inside the incubator 10 is controlled, and thus a gas concentration around the culture vessel 20 can be adjusted.

That is, the oxygen cylinder O and the carbon dioxide cylinder C are each connected to the incubator 10 via a valve, switching operations of the respective valves are controlled by the control device 60, and the gas supply to the incubator 10 from each gas cylinder is controlled, thus allowing the adjustment of oxygen concentration inside the incubator 10.

As the incubator 10, for example, a CO2 incubator is usable. As the placement table 11 inside the incubator 10, one made of a perforated metal or the like can be disposed to be used, and the culture vessel 20 is placed on the placement table 11 and housed in a closed manner. The placement table 11 may be omitted.

Since the cell culture system of this embodiment is applicable to a case where cells are cultured without using the incubator 10, the incubator 10 may be omitted.

Next, the culture vessel 20 in the cell culture system of this embodiment will be described in detail.

The culture vessel 20 is a cell culture vessel of a closed system that is at least partially formed of a gas permeable member and has a gas permeability, and for example, one formed by heat-sealing peripheral edge portions of two rectangular gas permeable films can be used.

The shape of the culture vessel 20 is formed to be inclined with respect to the ports in the vicinities of the ports, and this allows easily flowing the medium to the ports. The shape of the culture vessel 20 may be a rectangular shape such as an elongated rectangle. The number of the ports included in the culture vessel 20 is not limited to two, and may be one or three or more.

In the cell culture system of this embodiment, a fluorescent oxygen concentration sensor 21 is disposed on a culture surface inside the culture vessel 20. In the incubator 10, a fluorescent light emitting/receiving unit 22 is disposed to be able to emit and receive a fluorescent light to the fluorescent oxygen concentration sensor 21.

The fluorescent light emitting/receiving unit 22 is, for example, disposed under the placement table 11, and the fluorescent light emitting/receiving unit 22 can emit and receive a fluorescent light to the fluorescent oxygen concentration sensor 21 via the placement table 11 when it is transparent, or via a through hole provided to the placement table 11. The fluorescent light emitting/receiving unit 22 can be disposed to be in contact with the lower side of the culture vessel 20.

By controlling the fluorescent light emitting/receiving unit 22 by the control device 60, the oxygen concentration inside the culture vessel 20 can be measured.

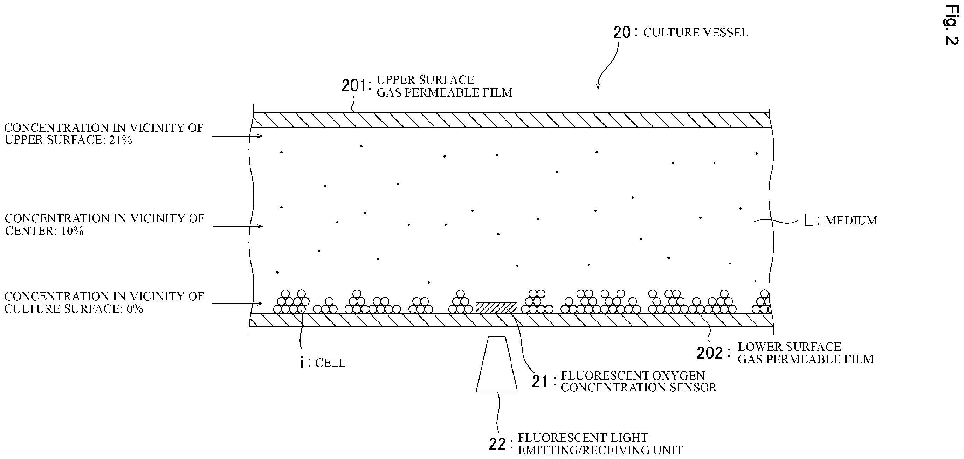

As illustrated in FIG. 2, the concentration of dissolved oxygen in the medium inside the culture vessel 20 largely differs depending on the position. That is, in the medium in the vicinity of the culture surface on which cells are present (upper surface of a lower surface gas permeable film 202), since the cells actively consume oxygen, oxygen is rapidly consumed, and gradually becomes 0% as the cell density increases. In contrast, while depending on the liquid thickness inside the culture vessel 20, for example, when the liquid thickness is 10 mm or more, in the medium in the vicinity of an upper surface (lower surface of an upper surface gas permeable film 201) in the opposite side of the culture surface inside the culture vessel 20, the oxygen concentration is slow in decrease from the initial state (for example, about 21% in atmosphere) when cells are not present in the vicinity. In the medium near the center of the culture vessel 20, the oxygen concentration is approximately middle (for example, 10%) of them.

Accordingly, in the cell culture system of this embodiment, by disposing the fluorescent oxygen concentration sensor 21 on the culture surface inside the culture vessel 20, the oxygen concentration inside the culture vessel 20 based on the oxygen consumption by the cells can be appropriately measured.

Additionally, as described later, in the cell culture system of this embodiment, by taking the oxygen permeability of the culture vessel 20 and the oxygen concentration around the culture vessel 20 into consideration, the oxygen consumption by the cells can be appropriately calculated.

Furthermore, according to the cell culture system of this embodiment, since the oxygen concentration inside the culture vessel 20 is continuously 0% to about 1%, even when the measurement of the oxygen concentration by the fluorescent oxygen concentration sensor 21 is difficult, the oxygen consumption by the cells can be appropriately estimated.

Here, a mechanism of gas permeation in the gas permeable film will be described.

The dissolved gas in the medium inside the culture vessel is dissolved at a concentration that equilibrates with a partial pressure around the culture vessel. The concentration of oxygen dissolved in the medium placed in the incubator is about 21% the same as the concentration in atmosphere when the gas concentration inside the incubator is not controlled. The consumption of the dissolved oxygen inside the culture vessel by the cells decreases the dissolved oxygen of the medium around the cells, and the oxygen gas permeates the film and flows into the culture vessel so as to keep the equilibrium with the oxygen concentration outside the culture vessel.

The gas permeability (speed) of the film is determined depending on the gas partial pressure difference between both sides of the film, affinity between the film and the gas, the diffusivity of gas molecules moving into the film, and the like.

Next, a principle of measuring the oxygen concentration by the fluorescent oxygen concentration sensor will be described.

When a light is applied to a fluorescent element of the fluorescent oxygen concentration sensor, a fluorescent substance absorbs the light and becomes in an excited state, and then returns to a ground state while emitting a fluorescent light. At this time, when oxygen is present around the fluorescent substance, the excitation energy is lost, and the fluorescence emission intensity decreases. Accordingly, the more oxygen is, the more the fluorescence emission intensity decreases. The fluorescent light emitting/receiving unit measures the oxygen amount based on the change of the emission intensity due to the number of oxygen molecules contacting the fluorescent element when oxygen dissolved in the medium contacts the fluorescent element.

As a material of the culture vessel 20, a resin film or the like can be preferably used, and for example, a polyolefin-based resin such as polyethylene and polypropylene is usable. For example, polyethylene, a copolymer of ethylene and α-olefin, a copolymer of ethylene and vinyl acetate, an ionomer using: a copolymer of ethylene and an acrylic acid or ethylene and a methacrylic acid; and a metal ion, and the like can be listed. Further, a polyolefin, a styrene-based elastomer, a polyester-based thermoplastic elastomer, and the like can be used. Furthermore, a soft vinyl chloride resin, a polybutadiene resin, an ethylene-vinyl acetate copolymer, a chlorinated polyethylene resin, a polyurethane-based thermoplastic elastomer, a polyester-based thermoplastic elastomer, a silicone-based thermoplastic elastomer, a styrene-based elastomer, such as styrene-butadiene-styrene (SBS), styrene-isoprene-styrene (SIS), styrene-ethylene-butylene-styrene (SEBS), and styrene-ethylene-propylene-styrene (SEPS), a polyolefin resin, a fluorine-based resin, and the like may be used.

As the gas permeable member used for at least a part of the culture vessel 20, among the above-described materials, especially, using a thermoplastic resin excellent in gas permeation performance is preferable, and a polyolefin-based resin, for example, polyethylene, such as LLDPE, or polypropylene can be preferably used. The gas permeable member is preferably a transparent material.

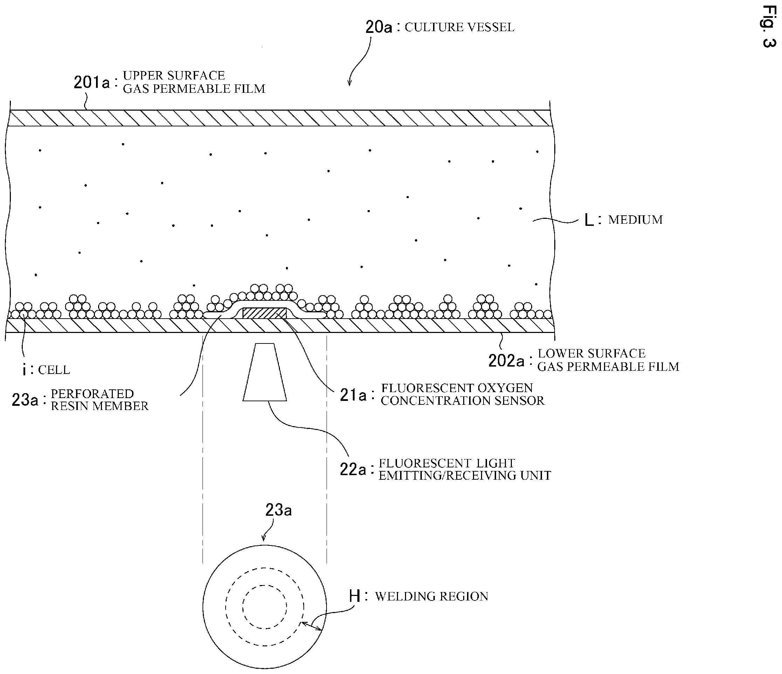

As illustrated in FIG. 3, as a modification of the cell culture system of this embodiment, it is preferred that a fluorescent oxygen concentration sensor 21a is secured to a culture surface (in the example of the drawing, upper surface of a lower surface gas permeable film 202a) inside a culture vessel 20a using a perforated resin member 23a. At this time, a fluorescent oxygen concentration sensor 21a is interposed between the perforated resin member 23a and the culture surface, and a welding region H of the perforated resin member 23a is welded to the culture surface, thereby allowing the securing.

As a material of the perforated resin member 23a, a polyolefin-based resin such as polyethylene, for example, LLDPE, and polypropylene is usable. For example, polyethylene, a copolymer of ethylene and α-olefin, a copolymer of ethylene and vinyl acetate, an ionomer using: a copolymer of ethylene and an acrylic acid or ethylene and a methacrylic acid; and a metal ion, and the like can be listed.

Since the perforated resin member 23a brings the fluorescent oxygen concentration sensor 21a into contact with the medium while securing the fluorescent oxygen concentration sensor 21a to the culture surface, a mesh material can be appropriately used. As the perforated resin member 23a, for example, a porous material such as a sponge can be used.

While the specific mesh material and porous material are not especially limited, one of a similar material having the melting point close to that of the material of the culture vessel 20a is preferable, and for example, a polyethylene mesh material (NBC Meshtec Inc., Item Number PE 200 me, or the like) and a polyethylene porous membrane (TEIJIN LIMITED, miraim (Trade name) large pore ≤ 3 μm) can be appropriately used.

In the cell culture system of this embodiment, with the configuration in which the fluorescent oxygen concentration sensor 21a is secured using the perforated resin member 23a, the removal and the move of the fluorescent oxygen concentration sensor 21a can be avoided when the medium is injected into the culture vessel 20a and the medium is discharged from the culture vessel 20a.

The cells cultured using the culture vessel 20 are not especially limited, and the cells may be floating cells, such as lymphocytes or dendritic cells, cultured by floating cells in a culture fluid, or may be adherent cells, such as induced pluripotent stem cells (iPS cells), neural stem cells, embryonic stem cells (ES cells), mesenchymal stem cells, hepatic cells, islet cells, cardiac muscle cells, corneal endothelial cells, and lymph corpuscles in an activation process, cultured by making cells adhere to a culture surface in the culture vessel.

The cells cultured using the culture vessel 20 may be formed in an aggregate such as a spheroid and an organoid, and the cell culture system and the method for detecting the proliferative property of cells of this embodiment are applied to the culture of an aggregate.

Next, the control device 60 in the cell culture system of this embodiment will be described in detail.

The control device 60 (control unit) includes, as illustrated in FIG. 1, an input/output unit 61, a fluorescent sensor input/output unit 62, a controller 63, an operation unit 64, and a power supply unit 65.

The input/output unit 61 is connected to the pump disposed between the culture vessel 20 and the medium supply container 40 and the pump disposed between the culture vessel 20 and the waste liquid container 50, and controls the operations of these pumps based on input information from the controller 63. Accordingly, the medium is supplied to the culture vessel 20 from the medium supply container 40, and the medium is discharged from the culture vessel 20 to the waste liquid container 50.

The input/output unit 61 is connected to a gas sensor 12 of the incubator 10, the valve disposed between the oxygen cylinder O and the incubator 10, and the valve disposed between the carbon dioxide cylinder C and the incubator 10, and transmits input information from the gas sensor 12 to the controller 63. The input/output unit 61 controls the operations of these valves based on the input information from the controller 63. Accordingly, the oxygen gas can be supplied from the oxygen cylinder O to the inside of the incubator 10, and the carbon dioxide gas can be supplied from the carbon dioxide cylinder C to the inside of the incubator 10, thus allowing the control of the gas concentration inside the incubator 10 to a desired level.

The fluorescent sensor input/output unit 62 is connected to the fluorescent light emitting/receiving unit 22, controls the fluorescent light emission of the fluorescent light emitting/receiving unit 22 based on input information from the controller 63, and transmits information based on the light reception from the fluorescent oxygen concentration sensor 21 to the controller 63. Accordingly, the oxygen concentration in the vicinity of the culture surface inside the culture vessel 20 can be measured, and the controller 63 can use this oxygen concentration for detecting the proliferative property of cells.

The controller 63 is configured of a PLC (programmable logic controller) or the like, can program and store a desired control content in advance, and can control the operations of the respective units based on it.

That is, the controller 63 transmits information for controlling the pumps and the valves to the input/output unit 61 at a predetermined timing. Additionally, based on any kinds of information including the input information from the fluorescent sensor input/output unit 62 and time information, information for controlling the pumps and the valves can be transmitted to the input/output unit 61.

The controller 63 includes a proliferation detection unit 631 that detects the proliferative property of cells inside the culture vessel 20.

The proliferation detection unit 631 detects the proliferative property of cells based on the oxygen permeability of the gas permeable member, the measurement value of the oxygen concentration sensor, the oxygen concentration around the culture vessel, and the oxygen consumption per cell of the cells. Specifically, the proliferation detection unit 631 can calculate the count of cells per unit area as the proliferative property of cells.

The oxygen permeability of the gas permeable member and the oxygen consumption per cell of the cells are calculated in advance corresponding to the usage, and stored in the proliferation detection unit 631 or the like.

As described later, as a result of calculating the oxygen consumption per cell of PBMC, it was 1.0e-9 mg/(hr cells). As a result of calculating the oxygen consumption per cell of Jurkat (lymphocyte cell line), it was 1.74e-9 mg/(hr·cells).

Prior to the determination of the oxygen permeability $(mg/(cm^2 \cdot hr \cdot atm))$, an actual culture configuration is determined first. That is, the oxygen permeability can be calculated as follows after determining the container size, the amount of the medium to be filled, the position of disposing the fluorescent sensor, and whether to use a pressing jig or not.

For example, it is assumed that a culture is performed using a culture bag used in Test 1 described later, which is formed by boning two polyethylene films having a thickness of 110 $\mu$m and has a bottom surface area of 50 $cm^2$, as the culture vessel 20 in a state where the fluorescent oxygen concentration sensor 21 is secured to the approximately center of the inner surface of this culture bag, and the culture vessel 20 is interposed between pressing jigs and pressurized. The bottom surface of the pressing jig is a transparent resin plate, and the fluorescent light emitting/receiving unit 22 is secured to a position corresponding to the fluorescent oxygen concentration sensor 21 in the resin plate.

Next, the medium is warmed to 37°C as a culture temperature, and injected into the culture vessel 20 together with the cells. As the cells, two kinds of PBMC (Peripheral Blood Mononuclear Cells) and Jurkat (lymphocyte cell line) were used, and the change amount of oxygen concentration was measured twice for each, thus calculating the oxygen permeability as follows and obtaining the average value.

As the medium for PBMC, one in which 2% of FBS was added to AlyS505N-7 (manufactured by Cell Science & Technology Institute, Inc.) was used, and 35 ml of the medium was filled in the culture bag together with the cells. As the medium for Jurkat, one in which 2% of FBS was added to AlyS505N-0 (manufactured by Cell Science & Technology Institute, Inc.) was used, and 35 ml of the medium was filled in the culture bag together with the cells.

The cells sank after approximately two hours from filling PBMC in the above-described culture bag with the cell filling amount per unit area of 2. 1e6 cells/cm$^2$, and the oxygen concentration stabilized. The oxygen concentration at that time was 6%.

In this case, the oxygen permeability G can be calculated as follows.

$$G = \text{cell filling amount per unit area} \times \text{oxygen consumption per cell/change}$$

$$\text{amount of oxygen concentration}$$

$$= 2.1\text{e}6 \times 1.0\text{e}{-}9/(21/100 - 6/100)$$

$$= 1.40\text{e}{-}2 \ (\text{mg}/(\text{cm}^2 \cdot \text{hr} \cdot \text{atm}))$$

After filling PBMC in the above-described culture bag with the cell filling amount per unit area of 4.0e6 cells/cm$^2$, the oxygen concentration around the culture bag was controlled to 30%. Approximately two hours later, the cells sank, and the oxygen concentration stabilized. The oxygen concentration at that time was 2%.
In this case, the oxygen permeability G can be calculated as follows.

$$G = \text{cell filling amount per unit area} \times \text{oxygen consumption per cell/change}$$

$$\text{amount of oxygen concentration}$$

$$= 4.0\text{e}6 \times 1.0\text{e}{-}9/(30/100 - 2/100)$$

$$= 1.43\text{e}{-}2 \ (\text{mg}/(\text{cm}^2 \cdot \text{hr} \cdot \text{atm}))$$

The cells sink after approximately two hours from filling Jurkat in the above-described culture bag with the cell filling amount per unit area of 1.1e6 cells/cm$^2$, and the oxygen concentration stabilizes. The oxygen concentration at that time was 8%.
In this case, the oxygen permeability G can be calculated as follows.

$$G = \text{cell filling amount per unit area} \times \text{oxygen consumption per cell/change}$$

$$\text{amount of oxygen concentration}$$

$$= 1.1\text{e}6 \times 1.74\text{e}{-}9/(21/100 - 8/100)$$

$$= 1.47\text{e}{-}2 \ (\text{mg}/(\text{cm}^2 \cdot \text{hr} \cdot \text{atm}))$$

After filling Jurkat in the above-described culture bag with the cell filling amount per unit area of 1.9e6 cells/cm$^2$, the oxygen concentration around the culture bag was controlled to 30%. Approximately two hours later, the cells sank, and the oxygen concentration stabilized. The oxygen concentration at that time was 5%.
In this case, the oxygen permeability G can be calculated as follows.

$$G = \text{cell filling amount per unit area} \times \text{oxygen consumption per cell/change}$$

$$\text{amount of oxygen concentration}$$

$$= 1.9\text{e}6 \times 1.74\text{e}{-}9/(30/100 - 5/100)$$

$$= 1.32\text{e}{-}2 \ (\text{mg}/(\text{cm}^2 \cdot \text{hr} \cdot \text{atm}))$$

From the above-described results, the oxygen permeability G of the culture bag used under the above-described conditions was determined as follows.

$$G = (1.40e-2 + 1.43e-2 + 1.47e-2 + 1.32e-2)/4$$

$$= 1.4e-2 \ (mg/(cm^2 \cdot hr \cdot atm))$$

The oxygen consumption per cell of the cells (mg/(hr·cells)) can be calculated as follows.

First, the cells and the medium are injected into a sealed vessel that does not permeate a gas in a state where the count of cells is obtained in advance. Air bubbles are discharged using a syringe or the like so as to obtain a state where air bubbles are not mixed in the sealed vessel as much as possible. The dissolved oxygen amount is calculated from the volume of the sealed vessel.

Specifically, the medium warmed to 37°C was filled in the sealed vessel having the volume of 10 ml. In the medium of 10 ml at 37°C (atmospheric oxygen concentration about 21%), oxygen of 6.86e-2 mg is dissolved (6.86e-2 mg/l@37°C (saturated dissolved oxygen)). The oxygen concentration change in the sealed vessel during the culture is measured, thus calculating the decrease in concentration of the dissolved oxygen per hour.

When PBMC is injected into the sealed vessel of 10 ml with the density of 2e6 cells/ml of the cell filling amount per unit area, 2e7 cells float in the vessel. At that time, the decrease of the oxygen concentration was 0.1 %/minute, and 6% per hour.

Since the decrease by 6% relative to 21% of the saturated dissolved oxygen corresponds to the decrease by 6/21 = 0.29, the oxygen consumption per unit time is as follows.

$$6.86e-2 \ mg \times 0.29 = 1.99e-2 \ mg/hr$$

Accordingly, the oxygen consumption S per cell (mg/(hr·cells)) of PBMC was calculated as follows.

$$S = 1.99e-2/2e7 = 1.0e-9 \ mg/(hr \cdot cells)$$

With the similar method, the oxygen consumption S per cell (mg/(hr·cells)) of Jurkat was calculated as follows.

$$S = 1.74e-9 \ mg/(hr \cdot cells)$$

The measurement value of the oxygen concentration sensor is the oxygen concentration in the vicinity of the culture surface inside the culture vessel 20 measured by the fluorescent oxygen concentration sensor 21, and input from the fluorescent light emitting/receiving unit 22 to the proliferation detection unit 631 via the fluorescent sensor input/output unit 62.

The oxygen concentration around the culture vessel is the oxygen concentration inside the incubator 10 in which the culture vessel 20 is housed. In a state where the oxygen concentration inside the incubator 10 is not controlled, or when the incubator 10 is not used, the oxygen concentration around the culture vessel is the atmospheric oxygen concentration (about 21%). This is set in advance by the controller 63.

Here, in the static culture, the amount of oxygen permeating the culture vessel matches and is balanced with the amount of oxygen consumed by the cells, and therefore, a formula below is satisfied.

$$G \times (M/100 - D/100) = C \times S$$

G: oxygen permeability of gas permeable member (mg/(cm$^2$·hr·atm))
M: oxygen concentration around culture vessel (%)
D: measurement value of oxygen concentration sensor (%)
C: count of cells per unit area (cells/cm$^2$)
S: oxygen consumption of one cell per unit time (mg/(hr - cells))

Accordingly, in the cell culture system of this embodiment, the proliferation detection unit 631 can calculate the count of cells per unit area as the proliferative property of cells by a formula (1) below.

$$C = G \times (M/100 - D/100)/S \ ... \ Formula \ (1)$$

C:  count of cells per unit area (cells/cm$^2$)
G:  oxygen permeability of gas permeable member (mg/(cm$^2$·hr·atm))
M:  oxygen concentration around culture vessel (%)
D:  measurement value of oxygen concentration sensor (%)
S:  oxygen consumption of one cell per unit time (mg/(hr·cells))

The count of cells per unit area calculated by the cell culture system of this embodiment is not one obtained by strictly measuring the actual count of cells, but one obtained by roughly estimating the count of cells based on the culture environment of the culture vessel 20.

In culturing cells in a large amount, since it is important to confirm whether the culture is properly performed or not, roughly estimating the count of cells inside the culture vessel is considerably beneficial.

In the cell culture system of this embodiment, it is also preferred that the proliferation detection unit 631 uses an estimated value of the oxygen concentration inside the culture vessel 20 at a desired time point obtained based on a ratio between a slope of a change in rise peak value of the oxygen concentration when an additional medium is added in the culture vessel 20 multiple times and a slope of a change of the oxygen concentration when the culture vessel 20 is stabilized as a measurement value D of the oxygen concentration sensor in the above-described formula (1).

That is, as described later in Test 5, when the cell density inside the culture vessel 20 becomes high because of the progress of the cell culture, the oxygen concentration in the vicinity of the culture surface inside the culture vessel 20 becomes less than 1% even when the oxygen concentration around the culture vessel 20 is increased to be high, and the measurement limit of the fluorescent oxygen concentration sensor 21 is exceeded, thus failing in the measurement with accuracy.

According to the cell culture system of this embodiment, even in such a case, by the above-described method, the count of cells per unit area inside the culture vessel 20 can be roughly estimated.

That is, by using an estimated value D' of the oxygen concentration inside the culture bag at a desired time point obtained based on a ratio between a slope of a change in rise peak value of the oxygen concentration when an additional medium is added in the culture bag multiple times and a slope of a change of the oxygen concentration when the culture bag is stabilized as the measurement value of the oxygen concentration sensor, the count of cells per unit area can be calculated based on a formula (2) below.

$$C = G \times (M/100 - D'/100)/S \ldots \text{Formula (2)}$$

The oxygen concentration at the rise peak of the oxygen concentration when the additional medium is added is influenced by the temperature of the medium to be added. Therefore, it is preferred to add the medium while holding the constant temperature.

The method for calculating the estimated value D' will be described in detail in Test 5.

The operation unit 64 includes a display unit such as a touch panel, and transmits input information by a user to the controller 63 to execute setting of PLC and the like. The operation unit 64 displays input information from the controller 63.

The power supply unit 65 (stabilized power source or the like) supplies electricity to the respective units in the control device 60.

Although not illustrated, the control device 60 can have a configuration further including a relaying unit (relay) and a circuit breaking unit (breaker). A part of or the whole of the configuration, for example, the controller 63 and the operation unit 64, of the control device 60 may be achieved by a microcomputer or a computer.

A method for detecting a proliferative property of cells of this embodiment is a method for detecting a proliferative property of cells in a cell culture statically culturing the cells by a culture vessel at least partially formed of a gas permeable member, and includes measuring an oxygen concentration in a vicinity of a culture surface inside the culture vessel by an oxygen concentration sensor disposed on the culture surface inside the culture vessel, and calculating a count of cells per unit area based on an oxygen permeability of the gas permeable member, a measurement value of the oxygen concentration sensor, an oxygen concentration around the culture vessel, and an oxygen consumption per cell of the cells.

In the method for detecting the proliferative property of cells of this embodiment, the count of cells per unit area can be calculated by the formula (1) below.

$$C = G \times (M/100 - D/100)/S \ldots \text{Formula (1)}$$

C:  count of cells per unit area (cells/cm$^2$)
G:  oxygen permeability of gas permeable member (mg/(cm$^2$·hr·atm))

M: oxygen concentration around culture vessel (%)
D: measurement value of oxygen concentration sensor (%)
S: oxygen consumption of one cell per unit time (mg/(hr·cells))

In the method for detecting the proliferative property of cells of this embodiment, it is also preferred that an estimated value of the oxygen concentration inside the culture vessel 20 at a desired time point obtained based on a ratio between a slope of a change in rise peak value of the oxygen concentration when an additional medium is added in the culture vessel 20 multiple times and a slope of a change of the oxygen concentration when the culture vessel 20 is stabilized is used as a measurement value D of the oxygen concentration sensor in the above-described formula (1).

As described above, according to the cell culture system and the method for detecting the proliferative property of cells of this embodiment, in the case where the cells are statically cultured with the culture vessel formed of the gas permeable member, the proliferative property of the cells inside the culture vessel at a desired timing during the culture can be detected, and the count of cells per unit area can be calculated as the proliferative property of the cells. Accordingly, this embodiment allows confirming whether the culture is properly performed or not in culturing cells in a large amount.

Examples

The following describes tests conducted for confirming the effects of the cell culture system and the method for detecting the proliferative property of cells according to the embodiments of the present invention.

In Test 1, Jurkat (lymphocyte cell line) was cultured to detect the proliferative property. In this test, the culture was continued without interruption during the test period, and the estimated value of the cell density was compared with the calculated value of the count of cells per unit area of this embodiment.

In Test 2, PBMC (Peripheral Blood Mononuclear Cells) were cultured, and the actually measured value of the cell density after the culture was compared with the calculated value of the count of cells per unit area of this embodiment.

In Test 3, PBMC were cultured, the culture was interrupted at a predetermined timing to count the actual count of cells, and the obtained actually measured value of the cell density was compared with the calculated value of the count of cells per unit area of this embodiment.

In Test 4, iPS cells were cultured, the actual count of cells was counted after the end of the culture, and the obtained actually measured value of the cell density was compared with the calculated value of the count of cells per unit area of this embodiment.

Test 5 is one conducted in a period following the culture of Test 3, and includes a period in which the cell density inside the culture vessel becomes high and the measurement value of the oxygen concentration in the vicinity of the culture surface is less than 1%. The actual count of cells was counted after the end of the culture, and the obtained actually measured value of the cell density was compared with the calculated value of the count of cells per unit area of this embodiment.

[Test 1]

As the culture bag, a culture bag (manufactured by Toyo Seikan Group Holdings, Ltd.) made of a linear low-density polyethylene having a thickness of 110 $\mu$m was prepared. The culture bag had an outer shape size of 120 mm $\times$ 65 mm, and a bottom surface area of the culture space of 48 cm$^2$. This culture bag was formed by heat-sealing peripheral edge portions of two flat films. This culture bag had the oxygen permeability of 1.4e-2 (mg/(cm$^2$·hr·atm)).

In the approximately center of the inner surface of this culture bag, a fluorescent oxygen concentration sensor (sensor chip PSt3 type, PreSens) was secured.

The culture was performed in a state where this culture bag was interposed between pressing jigs and pressurized. The bottom surface of the pressing jig was a transparent resin plate, and the fluorescent light emitting/receiving unit was secured to a position corresponding to the fluorescent oxygen concentration sensor in the resin plate. The fluorescent light emitting/receiving unit was attached to the fluorescent sensor input/output unit of the control device, and as the fluorescent sensor input/output unit, OXY-4mini (TAITEC CORPORATION) was used.

The used fluorescent oxygen concentration sensor, fluorescent light emitting/receiving unit, and fluorescent sensor input/output unit are similar in the following tests.

As the cells, Jurkat (cell line derived from human leukemia T cells) were seeded with the cell density (count of cells per unit area) of 1.20e6 cells/cm$^2$.

As the medium, one in which 2% of FBS was added to AlyS505N-0 (manufactured by Cell Science & Technology Institute, Inc.) was used, and 35 ml of the medium was filled in the culture bag together with the cells.

As the incubator, a multi-gas incubator capable of high oxygen culture (PHC Corporation, model number MCO-5M-PJ) was used, and an automatic liquid transfer mechanism was installed in the incubator.

A medium supply bag in which the medium was filled was stored in a cooler at 10°C, and connected to the culture bag by a tube. A waste liquid bag was connected to the culture bag by a tube. The medium was transferred by controlling a tube pump. The medium was warmed while passing through inside the incubator at 37°C, and became about 30°C at the point of entering the culture bag.

The control of the tube pump and the control of the oxygen concentration of the incubator were performed by the controller of the control device. In each test, the proliferation detection unit of the controller was configured by a computer.

Then, by automatically replacing a part of the medium at a designated time, the medium deteriorated by the culture was discharged from the culture bag, and a new medium was filled.

Specifically, the replacement of the medium was performed at a predetermined timing after the start of the culture. At this time, the medium discharge amount was 25 ml in 35 ml. The liquid transfer rate was 2 ml/minute. When a new medium was added by the replacement of the medium, the oxygen concentration inside the culture bag temporarily increased, and the peak was indicated in a graph described later.

The oxygen concentration of the incubator was increased at designated timings.

Specifically, the change from 21% to 25% was made at a timing after 23 hours from the start of the culture. The change from 25% to 30% was made at a timing after 32 hours from the start of the culture. The change from 30% to 35% was made at a timing after 50 hours from the start of the culture. The change from 35% to 40% was made at a timing after 53 hours from the start of the culture.

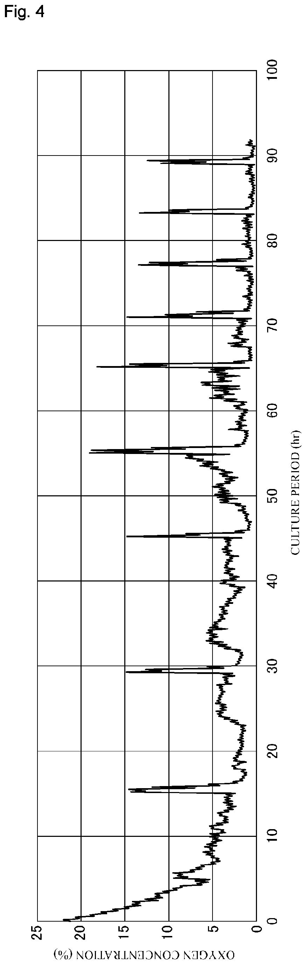

FIG. 4 illustrates a graph indicating the measurement result of the oxygen concentration by the fluorescent oxygen concentration sensor secured to the culture bag in the cell culture of Test 1.

Based on this measurement result, the estimated value of the cell density was compared with the calculated value of the count of cells per unit area of this embodiment after 10, 40, and 60 hours from the start of the culture.

Specifically, the proliferation speed of Jurkat cells is about 1.4 times/24hr, and relatively stable. Therefore, based on the cell density at the seeding (1.20e6 cells/cm$^2$), the cell densities after 24, 48 hours from the start of the culture were estimated as 1.68e6 cells/cm$^2$ (= 1.20e6 × 1.4), 2.35e6 cells/cm$^2$ (= 1.68e6 × 1.4), respectively, and based on them, the estimated values of the cell density after 10, 40, and 60 hours were calculated as follows.

$$\text{Cell density estimated value after 10 hours } 1.40\text{e6 cells/cm}^2 \; (= (1.20\text{e6} \times 1.4 - 1.20\text{e6}) \times 10/24 + 1.20\text{e6})$$

$$\text{Cell density estimated value after 40 hours } 2.13\text{e6 cells/cm}^2 \; (= (1.68\text{e6} \times 1.4 - 1.68\text{e6}) \times 16/24 + 1.68\text{e6})$$

$$\text{Cell density estimated value after 60 hours } 2.82\text{e6 cells/cm}^2 \; (= (2.35\text{e6} \times 1.4 - 2.35\text{e6}) \times 12/24 + 2.35\text{e6})$$

The calculated value of the count of cells per unit area in the cell culture system and the method for detecting the proliferative property of cells of this embodiment is, for after 10, 40, and 60 hours, calculated as follows. First, the oxygen permeability of the gas permeable member (mg/(cm$^2$·hr·atm)) was 1.4e-2. The oxygen consumption of one cell per unit time (mg/(hr·cells)) was 1.74e-9.

After 10 hours, the oxygen concentration (%) around the culture vessel was 21%, and the measurement value (%) of the oxygen concentration sensor was 5%.

Accordingly, a count C of cells per unit area (cells/cm$^2$) after 10 hours is as follows.

$$C = 1.4\text{e-}2 \times (21/100 - 5/100)/1.74\text{e-}9 = 1.29\text{e6}$$

In contrast, the cell density estimated value after 10 hours was, as described above, 1.40e6 cells/cm$^2$.

After 40 hours, the oxygen concentration (%) around the culture vessel was 30%, and the measurement value (%) of the oxygen concentration sensor was 3%.

Accordingly, the count C of cells per unit area (cells/cm$^2$) after 40 hours is as follows.

$$C = 1.4\text{e-}2 \times (30/100 - 3/100)/1.74\text{e-}9 = 2.17\text{e6}$$

In contrast, the cell density estimated value after 40 hours was, as described above, 2.13e6 cells/cm$^2$.

Furthermore, after 60 hours, the oxygen concentration (%) around the culture vessel was 40%, and the measurement value (%) of the oxygen concentration sensor was 2%.

Accordingly, the count C of cells per unit area (cells/cm$^2$) after 60 hours is as follows.

$$C = 1.4e{-}2 \times (40/100 - 2/100)/1.74e{-}9 = 3.06e6$$

In contrast, the cell density estimated value after 60 hours was, as described above, 2.82e6 cells/cm$^2$.

As described above, it was seen that according to the cell culture system and the method for detecting the proliferative property of cells of this embodiment, when the cells are statically cultured with the culture vessel formed of the gas permeable member, the count of cells per unit area at a desired timing during the culture can be roughly calculated.

[Test 2]

As the culture bag, the same one as Test 1 was prepared. This culture bag had the oxygen permeability of 1.4e-2 (mg/(cm$^2$·hr·atm)).

In the approximately center of the inner surface of this culture bag, a fluorescent oxygen concentration sensor was secured.

The culture was performed in a state where this culture bag was interposed between pressing jigs and pressurized. The bottom surface of the pressing jig was a transparent resin plate, and the fluorescent light emitting/receiving unit was secured to a position corresponding to the fluorescent oxygen concentration sensor in the resin plate.

As the cells, PBMC (Peripheral Blood Mononuclear Cells) were used. Prior to seeding in the culture bag, the PBMC were activated for three days using a flask that was coated with an anti-CD3 antibody and immobilized, and then, the PBMC were seeded in the culture bag with the cell density of 1.6e6 cells/cm$^2$.

As the medium, one in which 2% of FBS was added to AlyS505N-7 (manufactured by Cell Science & Technology Institute, Inc.) was used, and 35 ml of the medium was filled in the culture bag together with the cells.

The oxygen concentration was not measured for three days after the seeding in the culture bag, and the oxygen concentration inside the culture bag was measured for about 60 hr in the next three days.

As the incubator, a multi-gas incubator capable of high oxygen culture (PHC Corporation, model number MCO-5M-PJ) was used, and an automatic liquid transfer mechanism was installed in the incubator.

A medium supply bag in which the medium was filled was stored in a cooler at 10°C, and connected to the culture bag by a tube. A waste liquid bag was connected to the culture bag by a tube. The medium was transferred by controlling a tube pump.

Then, by automatically replacing a part of the medium at a designated time, the medium deteriorated by the culture was discharged from the culture bag, and a new medium was filled.

Specifically, the replacement of the medium was performed at a predetermined timing after the start of the culture. At this time, the medium discharge amount was 25 ml in 35 ml. The liquid transfer rate was 2 ml/minute. When a new medium was added by the replacement of the medium, the oxygen concentration inside the culture bag temporarily increased, and the peak was indicated in a graph described later.

In this test, the oxygen concentration of the incubator 10 was not increased, and the culture with high oxygen was not performed.

Then, after 61 hours from the measurement start, the cells were uniformly floated in the medium by stirring inside the culture bag, the medium was extracted from the culture bag by about 0.5 ml using a syringe, and the cells were stained by trypan blue, thus measuring the cell density using a hemocytometer. As a result, the cell density after 61 hours from the measurement start was 2.34e6 cells/cm$^2$.

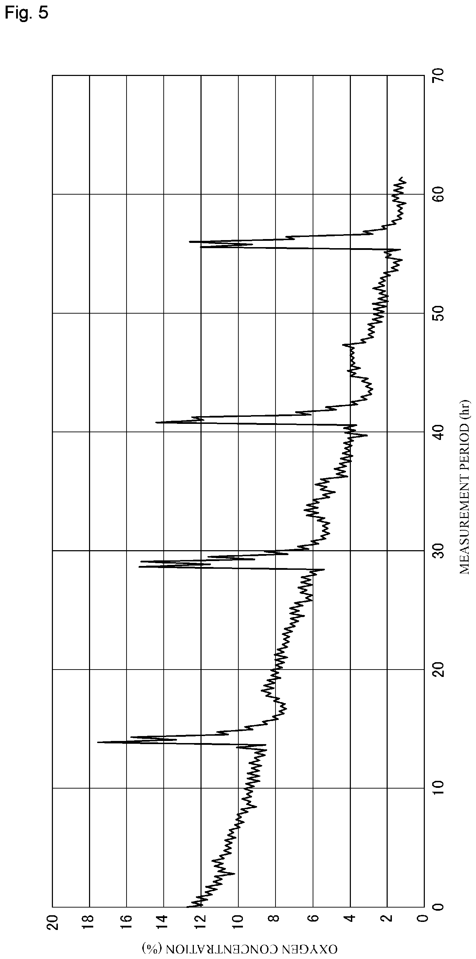

FIG. 5 illustrates a graph indicating the measurement result of the oxygen concentration by the fluorescent oxygen concentration sensor secured to the culture bag in the cell culture of Test 2.

The calculated value of the count of cells per unit area in the cell culture system and the method for detecting the proliferative property of cells of this embodiment is, for after 61 hours, calculated as follows.

First, the oxygen permeability of the gas permeable member (mg/(cm$^2$·hr·atm)) was 1.4e-2. The oxygen consumption of one cell per unit time (mg/(hr·cells)) was 1.00e-9.

The oxygen concentration (%) around the culture vessel was 21%, and the measurement value (%) of the oxygen concentration sensor was 1.2%.

Accordingly, the count C of cells per unit area (cells/cm$^2$) is as follows.

$$C = 1.4e{-}2 \times (21/100 - 1.2/100)/1.00e{-}9 = 2.77e6$$

In contrast, the actually measured value of the cell density was, as described above, 2.34e6 cells/cm². **[0161]** That is, also by this test, it was seen that according to the cell culture system and the method for detecting the proliferative property of cells of this embodiment, when the cells are statically cultured with the culture vessel formed of the gas permeable member, the count of cells per unit area can be roughly calculated.

[Test 3]

As the culture bag, the same one as Test 1 was prepared. This culture bag had the oxygen permeability of 1.4e-2 (mg/(cm²·hr·atm)).

In the approximately center of the inner surface of this culture bag, a fluorescent oxygen concentration sensor was secured.

The culture was performed in a state where this culture bag was interposed between pressing jigs and pressurized. The bottom surface of the pressing jig was a transparent resin plate, and the fluorescent light emitting/receiving unit was secured to a position corresponding to the fluorescent oxygen concentration sensor in the resin plate.

As the cells, PBMC (Peripheral Blood Mononuclear Cells) were used. Prior to seeding in the culture bag, the PBMC were activated for three days using a flask that was coated with an anti-CD3 antibody and immobilized, and then, the PBMC were seeded in the culture bag with the cell density of 1.06e6 cells/cm².

As the medium, one in which 2% of FBS was added to AlyS505N-7 (manufactured by Cell Science & Technology Institute, Inc.) was used, and 35 ml of the medium was filled in the culture bag together with the cells.

The oxygen concentration was not measured for four days after the seeding in the culture bag, and the oxygen concentration inside the culture bag was measured for about 65 hr in the next three days.

However, in Test 3, different from Test 2, for measuring the count of cells, the automatic liquid transfer was stopped, the cells were uniformly floated in the medium by stirring inside the culture bag, the medium was extracted from the culture bag by about 0.1 ml using a syringe, and the cells were stained by trypan blue, thus measuring the cell density using a hemocytometer. The number of extracted cells was extremely small, and does not affect the cell density of the whole.

The use of the incubator and the replacement of the medium were performed similarly to Test 1.

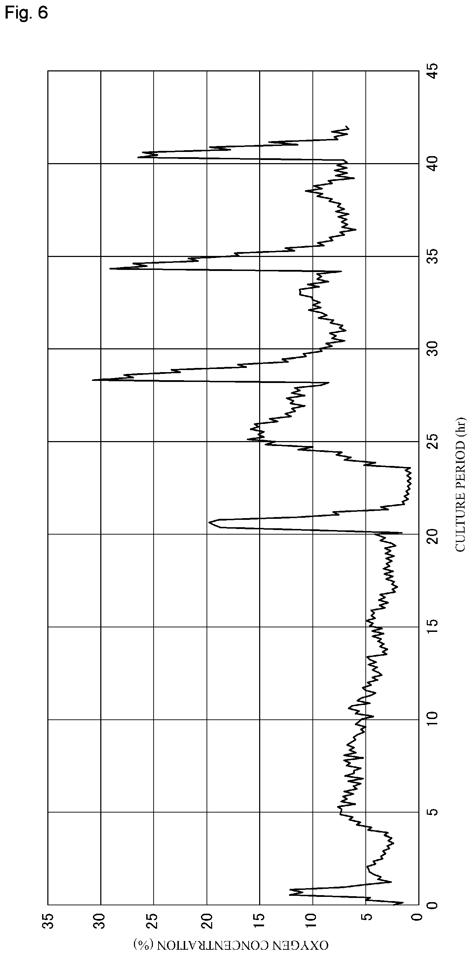

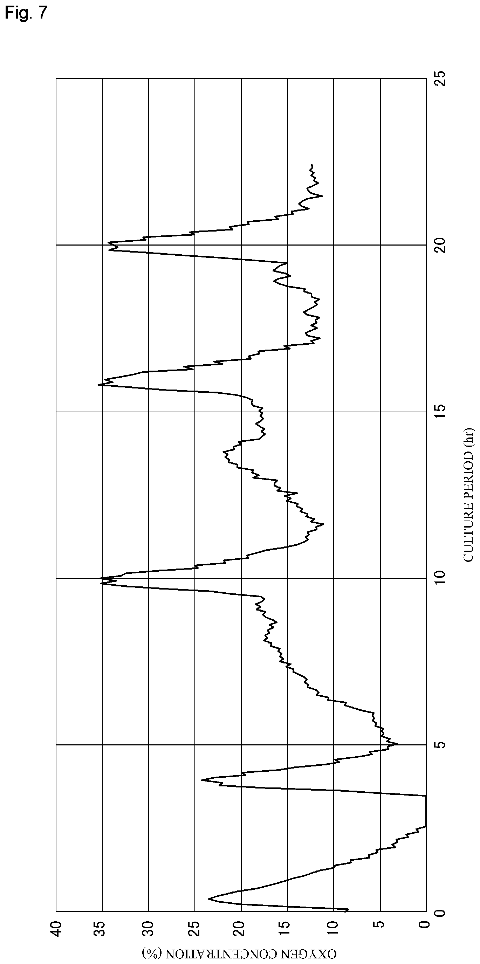

FIG. 6 and FIG. 7 illustrate graphs indicating the measurement result of the oxygen concentration by the fluorescent oxygen concentration sensor secured to the culture bag in the cell culture of Test 3. The graph of FIG. 6 indicates the measurement result of the oxygen concentration up to the first measurement of the count of cells, and the graph of FIG. 7 indicates the measurement result of the oxygen concentration up to the second measurement of the count of cells.

In this test, the oxygen concentration of the incubator 10 was increased at designated timings.

Specifically, in the graph of FIG. 6, the change from 21% to 30% was made at a timing after 4 hours from the measurement start. The change from 30% to 40% was made at a timing after 23 hours from the start of the measurement. Further, in the graph of FIG. 7, the change from 40% to 50% was made at a timing after 6 hours from the start of the measurement.

Then, in the graph of FIG. 6, after 42 hours from the measurement start, the cells were uniformly floated in the medium by stirring inside the culture bag, the medium was extracted from the culture bag by about 0.1 ml, and the cells were stained by trypan blue, thus measuring the cell density using a hemocytometer. As a result, the cell density after 42 hours from the measurement start was 4.04e6 cells/cm².

Then, in the graph of FIG. 7, after 22.5 hours from the measurement start, the cells were uniformly floated in the medium by stirring inside the culture bag, the medium was extracted from the culture bag by about 0.1 ml, and the cells were stained by trypan blue, thus measuring the cell density using a hemocytometer. As a result, the cell density after 22.5 hours from the measurement start was 5.2e6 cells/cm².

The calculated value of the count of cells per unit area in the cell culture system and the method for detecting the proliferative property of cells of this embodiment is calculated as follows.

First, the oxygen permeability of the gas permeable member (mg/(cm²·hr·atm)) was 1.4e-2. The oxygen consumption of one cell per unit time (mg/(hr·cells)) was 1.00e-9.

After 42 hours from the measurement start in the graph of FIG. 6, the oxygen concentration (%) around the culture vessel was 40%, and the measurement value (%) of the oxygen concentration sensor was 7%.

Accordingly, the count C of cells per unit area (cells/cm²) is as follows.

$$C = 1.4e{-}2 \times (40/100 - 7/100)/1.00e{-}9 = 4.62e6$$

In contrast, the actually measured value of the cell density was, as described above, 4.04e6 cells/cm².

After 22.5 hours from the measurement start in the graph of FIG. 7, the oxygen concentration (%) around the culture vessel was 50%, and the measurement value (%) of the oxygen concentration sensor was 12%.

Accordingly, the count C of cells per unit area (cells/cm$^2$) is as follows.

$$C = 1.4e{-}2 \times (50/100 - 12/100)/1.00e{-}9 = 5.32e6$$

In contrast, the actually measured value of the cell density was, as described above, 5.2e6 cells/cm$^2$.

That is, also by this test, it was seen that according to the cell culture system and the method for detecting the proliferative property of cells of this embodiment, when the cells are statically cultured with the culture vessel formed of the gas permeable member, the count of cells per unit area can be roughly calculated.

[Test 4]

As the culture bag, a culture bag (manufactured by Toyo Seikan Group Holdings, Ltd.) made of a linear low-density polyethylene having a thickness of 110 $\mu$m was prepared. The culture bag had an outer shape size of 120 mm $\times$ 65 mm, and a bottom surface area of the culture space of 48cm$^2$.

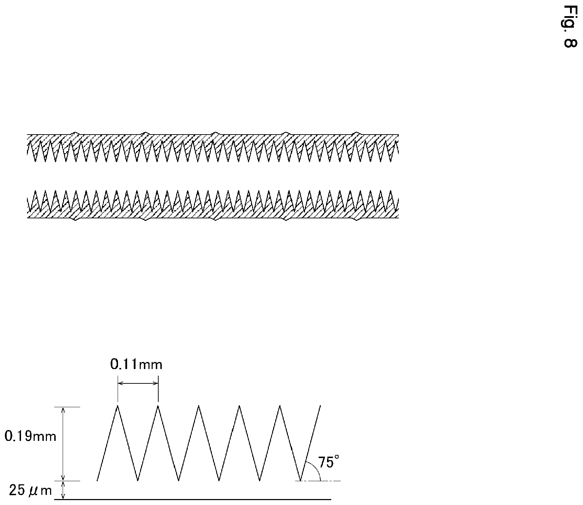

As illustrated in FIG. 8, this culture bag is provided with a plurality of protrusions inside, and a plurality of small protrusions formed on an outer surface. Specifically, this culture bag was formed by heat-sealing peripheral edge portions of two films in which a plurality of approximately triangular prisms, provided with a pitch of 0.11 mm, a height of 0.19 mm, and an angle of 75°, were processed to be arranged in parallel in a mountain range pattern without intervals. The thickness of the thinnest portion (thin portion) of the film is 25 $\mu$m. This culture bag had the oxygen permeability of 8.0e-2 (mg/(cm$^2$·hr·atm)). The oxygen permeability of this culture bag was calculated similarly to the method described with the example of the culture bag used in Test 1.

In the approximately center of the inner surface of this culture bag, a fluorescent oxygen concentration sensor was secured.

In this test, the culture was performed without using the pressing jigs. The fluorescent light emitting/receiving unit was disposed at a position corresponding to the fluorescent oxygen concentration sensor in the lower side of the culture bag.

As the cells, iPS cells (cell line 1231A3, Center for iPS Cell Research and Application, Kyoto University) were seeded with the cell density of 1.7e4 cells/cm$^2$. In this test, both of the upper surface and the lower surface inside the culture bag are used as culture surfaces, and this cell density includes both surfaces.

As the medium, StemFit(R) (AK-02N, Ajinomoto Healthy Supply Co., INC.) was used, and 35 ml of the medium was filled in the culture bag together with the cells.

As the incubator, a multi-gas incubator capable of high oxygen culture (PHC Corporation, model number MCO-5M-PJ) was used. In this test, the automatic liquid transfer mechanism was not used, and the replacement of the medium was manually performed.

A medium supply bag in which the medium was filled was stored in a cooler at 10°C, and connected to the culture bag by a tube. A waste liquid bag was connected to the culture bag by a tube. The medium was transferred by controlling a tube pump. The medium was warmed while passing through inside the incubator at 37°C, and became about 30°C at the point of entering the culture bag.

Then, by replacing the whole amount of the medium at a designated time, the medium deteriorated by the culture was discharged from the culture bag, and a new medium was filled.

Specifically, the replacement of the medium was performed at a predetermined timing after the start of the culture. At this time, the medium discharge amount was 35 ml in 35 ml, and the liquid transfer rate was 2 ml/minute. When a new medium was added by the replacement of the medium, the oxygen concentration inside the culture bag temporarily increased, and the peak was indicated in a graph described later.

In this test, the oxygen concentration of the incubator 10 was not increased, and the culture with high oxygen was not performed.

Then, after the medium was removed from the culture bag after 63 hours from the measurement start, the cells were detached from the culture bag using a cell detachment solution (Thermo Fisher Scientific K.K., TrypLE). Then, the medium was filled in the culture bag, the detached cells were suspended in the medium, the whole amount of the medium was collected from the culture bag using a syringe, and the cells were stained by trypan blue, thus measuring the cell density using a hemocytometer. As a result, the cell density was 3.20e6 cells/cm$^2$.

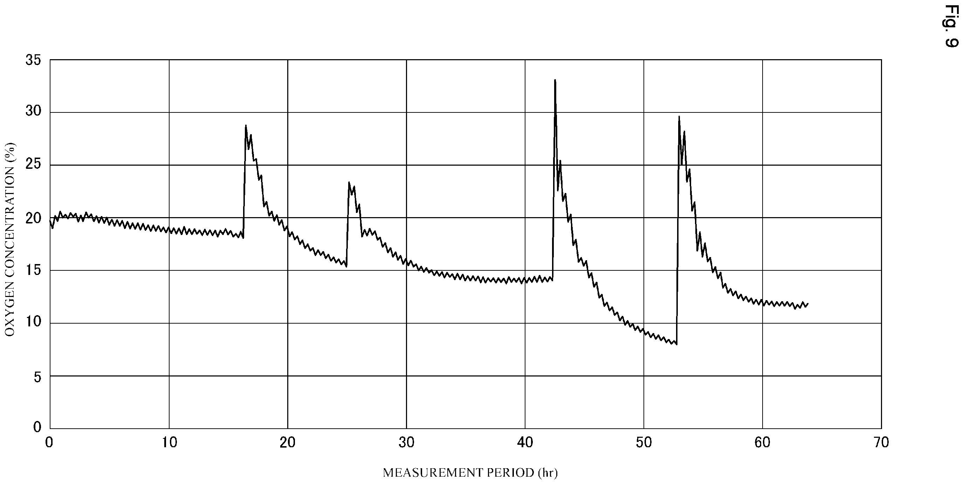

FIG. 9 illustrates a graph indicating the measurement result of the oxygen concentration by the fluorescent oxygen concentration sensor secured to the culture bag in the cell culture of Test 4.

The calculated value of the count of cells per unit area in the cell culture system and the method for detecting the proliferative property of cells of this embodiment is calculated as follows.

First, the oxygen permeability of the gas permeable member (mg/(cm$^2$·hr·atm)) was 8.0e-2. The oxygen consumption of one cell per unit time (mg/(hr·cells)) was 2.34e-9.

The oxygen concentration (%) around the culture vessel was 21%, and the measurement value (%) of the oxygen concentration sensor was 12%.

Accordingly, the count C of cells per unit area (cells/cm$^2$) is as follows.

$$C = 8.0e{-}2 \times (21/100 - 12/100)/2.34e{-}9 = 3.08e6$$

In contrast, the actually measured value of the cell density was, as described above, 3.20e6 cells/cm$^2$.

That is, also by this test, it was seen that according to the cell culture system and the method for detecting the proliferative property of cells of this embodiment, when the cells are statically cultured with the culture vessel formed of the gas permeable member, the count of cells per unit area can be roughly calculated.

[Test 5]

This test was sequentially conducted after Test 3, and the oxygen concentration inside the culture bag was measured for about 60 hr in three days subsequent to the oxygen concentration measurement in Test 3.

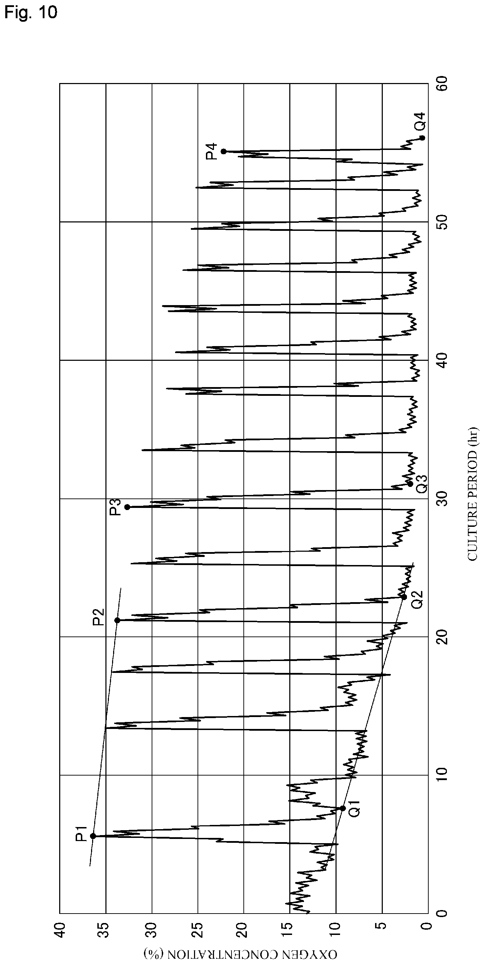

FIG. 10 illustrates a graph indicating the measurement result of the oxygen concentration by the fluorescent oxygen concentration sensor secured to the culture bag in the cell culture of Test 5.

After 56 hours from the measurement start, the cells were uniformly floated in the medium by stirring inside the culture bag, the medium was extracted from the culture bag by about 0.1 ml, and the cells were stained by trypan blue, thus measuring the cell density using a hemocytometer. As a result, the cell density after 56 hours from the measurement start was 1.04e7 cells/cm$^2$.

In this test, as illustrated in FIG. 10, from 25 hours after the measurement start, the cell density inside the culture bag is very high. Especially, at the point of 56 hours after the measurement start, the oxygen concentration inside the culture bag is less than 1%.

In such a case, in the cell culture system and the method for detecting the proliferative property of cells of this embodiment, the count of cells per unit area is calculated by the following method.

First, in a culture period before the cell density inside the culture bag becomes very high, a slope PT of the change in rise peak value of the oxygen concentration when the additional medium is added in the culture bag multiple times, and a slope QT of the change of the oxygen concentration when the culture bag is stabilized are calculated, and the ratio (QT/PT) is calculated.

Specifically, in FIG. 10, the slope PT of the change in rise peak value between P1 (5.5, 36.3) and P2 (20.1, 34.1) is -15% (= (34.1 - 36.3)/(20.1 - 5.5) × 100), the slope QT of the change of the oxygen concentration between Q1 (7.7, 9.3) and Q2 (22.9, 2.6) is -44% (= (2.6 - 9.3)/(22.9 - 7.7) × 100), and the ratio (QT/PT) is 2.9.

Next, the peak immediately before Q4 (56, less than 1) after 56 hours from the measurement start is P4 (55.1, 22.3), and the peak immediately before Q3 (31.1, 2.3) at which the oxygen concentration can be measured is P3 (29.5, 32.7).

It can be estimated that the oxygen concentration at Q4 changes from Q3 based on the above-described ratio (QT/PT) relative to the change amount of the oxygen concentration from P3 to P4.

Accordingly, an oxygen concentration D' to be estimated can be calculated as follows.

$$D' = \text{oxygen concentration at Q3} - (\text{change amount of oxygen concentration}$$

$$\text{from P3 to P4}) \times QT/PT$$

That is, by using the estimated value D' of the oxygen concentration inside the culture bag at a desired time point obtained based on the ratio between the slope of the change in rise peak value of the oxygen concentration when the additional medium is added in the culture bag multiple times and the slope of the change of the oxygen concentration when the culture bag is stabilized as the measurement value of the oxygen concentration sensor, the count of cells per unit area can be calculated based on a formula (2) below.

$$C = G \times (M/100 - D'/100)/S \dots \text{Formula (2)}$$

Accordingly, according to the cell culture system and the method for detecting the proliferative property of cells of this embodiment, the count of cells per unit area can be calculated as follows.

The oxygen permeability of the gas permeable member (mg/(cm$^2$·hr·atm)) was 1.4e-2. The oxygen consumption of one cell per unit time (mg/(hr·cells)) was 1.00e-9.

$$D' = 2.3 - (22.3 - 32.7) \times 2.9$$

$$= -27.9$$

$$C = 1.4e{-}2 \times (50/100 + 27.9/100)/1.00e{-}9 = 1.09e7 \text{ cells/cm}^2$$

In contrast, the actually measured value of the cell density was, as described above, 1.04e7 cells/cm$^2$.

That is, also by this test, it was seen that according to the cell culture system and the method for detecting the proliferative property of cells of this embodiment, when the cells are statically cultured with the culture vessel formed of the gas permeable member, the count of cells per unit area can be roughly calculated.

The present invention is not limited to the above-described embodiments and examples, and, needless to say, various modifications can be made within the scope of the present invention. For example, the culture vessel is not limited to the exemplified one, and one appropriately changed corresponding to the purpose of the culture, for example, one including one port or three ports, and one provided with a plurality of wells on the culture surface, can be used.

INDUSTRIAL APPLICABILITY

The present invention can be appropriately used, for example, when cells are cultured in a large amount with a high density using a cell culture bag.

The entire contents of documents mentioned in this specification and the specification of the Japanese patent application to which this application claims priority under the Paris Convention are entirely incorporated by reference herein.

DESCRIPTION OF REFERENCE SIGNS

| | |
|---|---|
| 10 | Incubator |
| 11 | Placement table |
| 12 | Gas sensor |
| 20, 20a | Culture vessel |
| 201, 201a | Upper surface gas permeable film |
| 202, 202a | Lower surface gas permeable film |
| 21, 21a | Fluorescent oxygen concentration sensor |
| 22, 22a | Fluorescent light emitting/receiving unit |
| 23a | Perforated resin member |
| 30 | Cooler |
| 40 | Medium supply container |
| 50 | Waste liquid container |
| 60 | Control device |
| 61 | Input/output unit |
| 62 | Fluorescent sensor input/output unit |
| 63 | Controller |
| 631 | Proliferation detection unit |
| 64 | Operation unit |
| 65 | Power supply unit |
| H | Welding region |
| i | Cell |
| L | Medium |
| O | Oxygen cylinder |
| C | Carbon dioxide cylinder |

**Claims**

1.  A cell culture system for statically culturing cells with a culture vessel, the culture vessel being at least partially formed of a gas permeable member, the cell culture system comprising:

    an oxygen concentration sensor that measures an oxygen concentration in a vicinity of a culture surface inside the culture vessel; and
    a proliferation detection unit that detects a proliferative property of the cells based on an oxygen permeability of the gas permeable member, a measurement value of the oxygen concentration sensor, an oxygen concentration around the culture vessel, and an oxygen consumption per cell of the cells.

2.  The cell culture system according to claim 1, wherein
    the proliferation detection unit calculates a count of the cells per unit area as the proliferative property of the cells by a formula (1) below,

$$C = G \times (M/100 - D/100)/S \dots \text{Formula (1)}$$

    C: count of cells per unit area (cells/cm$^2$)
    G: oxygen permeability of gas permeable member (mg/(cm$^2$·hr·atm))
    M: oxygen concentration around culture vessel (%)
    D: measurement value of oxygen concentration sensor (%)
    S: oxygen consumption of one cell per unit time (mg/(hr cells)).

3.  The cell culture system according to claim 1 or 2, wherein
    the proliferation detection unit uses an estimated value of an oxygen concentration inside the culture vessel at a desired time point as the measurement value of the oxygen concentration sensor, and the estimated value of the oxygen concentration inside the culture vessel at a desired time point is obtained based on a ratio between a slope of a change in rise peak value of the oxygen concentration when an additional medium is added in the culture vessel multiple times and a slope of a change of the oxygen concentration when the culture vessel is stabilized.

4.  The cell culture system according to any one of claims 1 to 3, wherein
    the oxygen concentration around the culture vessel is an oxygen concentration inside an incubator in which the culture vessel is housed, or an atmospheric oxygen concentration.

5.  The cell culture system according to any one of claims 1 to 4, wherein
    the oxygen concentration sensor is a fluorescent type.

6.  The cell culture system according to any one of claims 1 to 5, wherein
    the oxygen concentration sensor is secured to the culture surface inside the culture vessel using a perforated resin member.

7.  The cell culture system according to any one of claims 1 to 6, wherein
    the culture vessel and the perforated resin member are made of any material selected from polyethylene, polypropylene, a copolymer of ethylene and α-olefin, a copolymer of ethylene and vinyl acetate, and an ionomer using: a copolymer of ethylene and an acrylic acid or ethylene and a methacrylic acid; and a metal ion.

8.  A method for detecting a proliferative property of cells in a cell culture in which the cells are statically cultured with a culture vessel, the culture vessel being at least partially formed of a gas permeable member, the method comprising:

    measuring an oxygen concentration in a vicinity of a culture surface inside the culture vessel by an oxygen concentration sensor disposed on the culture surface inside the culture vessel; and
    calculating a count of the cells per unit area based on an oxygen permeability of the gas permeable member, a measurement value of the oxygen concentration sensor, an oxygen concentration around the culture vessel, and an oxygen consumption per cell of the cells.

Fig. 1

Fig. 2

EP 4 245 836 A1

20 : CULTURE VESSEL

201 : UPPER SURFACE GAS PERMEABLE FILM

CONCENTRATION IN VICINITY OF UPPER SURFACE: 21%

CONCENTRATION IN VICINITY OF CENTER: 10%

L : MEDIUM

CONCENTRATION IN VICINITY OF CULTURE SURFACE: 0%

i : CELL

202 : LOWER SURFACE GAS PERMEABLE FILM

21 : FLUORESCENT OXYGEN CONCENTRATION SENSOR

22 : FLUORESCENT LIGHT EMITTING/RECEIVING UNIT

Fig. 3

Fig. 4

Fig. 5

Fig. 6

Fig. 7

CULTURE PERIOD (hr)

OXYGEN CONCENTRATION (%)

Fig. 8

Fig. 9

Fig. 10

## INTERNATIONAL SEARCH REPORT

| International application No. |
| --- |
| **PCT/JP2021/041461** |

**A.    CLASSIFICATION OF SUBJECT MATTER**

*C12M 1/00*(2006.01)i; *C12M 1/04*(2006.01)i; *C12M 1/34*(2006.01)i; *C12M 3/00*(2006.01)i; *C12Q 1/02*(2006.01)i
FI:    C12M1/00 C; C12M1/04; C12M1/34 D; C12Q1/02; C12M3/00 Z

According to International Patent Classification (IPC) or to both national classification and IPC

**B.    FIELDS SEARCHED**

Minimum documentation searched (classification system followed by classification symbols)

C12M1/00; C12M1/04; C12M1/34; C12M3/00; C12Q1/02

Documentation searched other than minimum documentation to the extent that such documents are included in the fields searched

Published examined utility model applications of Japan 1922-1996
Published unexamined utility model applications of Japan 1971-2021
Registered utility model specifications of Japan 1996-2021
Published registered utility model applications of Japan 1994-2021

Electronic data base consulted during the international search (name of data base and, where practicable, search terms used)

JSTPlus/JMEDPlus/JST7580 (JDreamIII)

**C.    DOCUMENTS CONSIDERED TO BE RELEVANT**

| Category* | Citation of document, with indication, where appropriate, of the relevant passages | Relevant to claim No. |
| --- | --- | --- |
| Y | JP 2015-516175 A (WILSON WOLF MANUFACTURING CORPORATION) 11 June 2015 (2015-06-11)<br>claims 1-15, example 10 | 1-8 |
| Y | US 2019/0218492 A1 (YALE UNIVERSITY) 18 July 2019 (2019-07-18)<br>claims 1-39, paragraph [0066], fig. 2A, 3A, 8 | 1-8 |

☐ Further documents are listed in the continuation of Box C.     ☑ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
| --- | --- |
| **23 December 2021** | **11 January 2022** |

| Name and mailing address of the ISA/JP | Authorized officer |
| --- | --- |
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

| International application No. |
| --- |
| **PCT/JP2021/041461** |

| Patent document cited in search report | | | Publication date (day/month/year) | Patent family member(s) | | | Publication date (day/month/year) |
| --- | --- | --- | --- | --- | --- | --- | --- |
| JP | 2015-516175 | A | 11 June 2015 | WO | 2013/173835 | A1 | |
| | | | | claims 1-15, example 10 | | | |
| | | | | EP | 2850175 | A1 | |
| | | | | US | 2015/0175966 | A1 | |
| US | 2019/0218492 | A1 | 18 July 2019 | WO | 2018/052834 | A1 | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP S6315150 A

- JP H6121667 A